# EUROPEAN PATENT SPECIFICATION

(11) **EP 4 037 624 B1**
(45) Date of publication and mention of the grant of the patent: **09.12.2026**
(21) Application number: 20788705.0
(22) Date of filing: 02.10.2020
(51) Int. Cl.: A61F 5/445, A61F 13/00, A61F 5/443

(54) **A MEDICAL DEVICE SYSTEM**
MEDIZINGERÄTESYSTEM
SYSTÈME DE DISPOSITIF MÉDICAL

(30) Priority: 04.10.2019 DK PA201970617
(43) Date of publication of application: 10.08.2022
(73) Proprietor: Coloplast A/S, 3050 Humlebaek (DK)
(72) Inventor: SPEIERMANN, Finn, 2830 Virum (DK); KNÖDLER, Stephanie, 2990 Nivaa (DK); WINDEBALLE, Lars, Stendevad, 2830 Virum (DK)
(86) International application number: PCT/DK2020/050266
(87) International publication number: WO 2021/063466

(56) References cited:
- WO-A1-2007/098762
- WO-A1-2013/184784
- WO-A1-2019/120428
- US-A1- 2009 030 382
- US-B1- 6 171 289
- US-B2- 9 020 601

## Description

The present disclosure relates to a medical device system. Particularly, the present disclosure relates to a medical device system comprising a medical device and a base station for the medical device.

### BACKGROUND

Medical devices in general are often exposed to harsh environments, including, but not limited to, body fluids (e.g., stomal output or exudate from a wound) and continued wear causing degradation of the medical device. Examples of such medical devices include ostomy appliances and wound dressings, and related devices, such as electronic medical devices configured to monitor such an ostomy appliance or wound dressing.

Stomal output often contains body fluids and visceral contents that are aggressive to both the skin of a user and to ostomy devices, these have a detrimental effect on the efficiency and integrity of the adhesive materials that are applied to attach the ostomy device to the user's skin surface. For users in general, safe, reliable and efficient ostomy devices are evidently highly desirable.

However, a particularly major and persistent concern of a large population of ostomists continues to be failure of the base plate adhesive attaching the ostomy appliance to the user's skin surface, because such failure almost inevitably leads to embarrassing and stigmatising leakage incidents. Such incidents in turn are known from several user interviews to lead to a reduced quality-of-life feeling. Adhesive failure of the base plate adhesive can result from various reasons. Most often, a leakage incident is caused by stomal output entering the interface formed between the proximal surface of the base plate and the user's skin, e.g. due to less-than-optimal attachment of the base plate to the skin arising from e.g. uneven skin surface or skin folds. This undesirable progression of stomal output "underneath" the adhesive leads to deterioration and/or weakening of the adhesive material carrying the weight and providing the seal of the ostomy appliance. Often such failure happens surprisingly fast and is only detectable for the user once the failure has already become so severe that leakage occurs, requiring immediate change of the ostomy appliance and possibly also of the user's clothes.

In other instances, the primary factor of adhesive failure is simply a question of how much time has elapsed since the base plate of the ostomy appliance was first applied to the user's skin surface. In addition to the output from the stoma itself, the peristomal skin surface continuously secretes some moisture (e.g. sweat). To mitigate this, most often adhesives of base plates for ostomy devices include hydrocolloid materials which are capable of absorbing high levels of moisture, thereby stabilizing the polymer matrix of the adhesive material and prolonging the lifetime ("wear time") of the base plate. However, eventually, the adhesion capability of the base plate cannot support the force exerted on the base plate from the load of the output collecting bag, and the appliance must be replaced.

As there can be considerable differences in the severity and/or speed by which adhesive failure and potentially leakage occur, which differences at least to some extent are correlated to various factors including those presented above, a mere indication that failure or leakage is imminent, or that it has already occurred, fails to represent a reliable and satisfactory solution to the problem of avoiding sudden embarrassing and stigmatising leakage incidents in ostomy appliances. In other words, the users of ostomy appliances could greatly benefit from an appliance solution which provides them with better guidance and options regarding how and - not least - how quickly to react to a beginning failure of or leakage from the adhesive of the base plate of the appliance. More generally, ostomists and health care professionals alike would welcome improvements in ostomy devices to reduce or eliminate the occurrence of sudden leakage incidents.

Many of the above-discussed aspects are applicable to wound dressings and users thereof, where in particular exudate amount, pattern, and propagation are of interest. By monitoring such exudate amounts, patterns, or propagation, the use of the wound dressing can be optimised, such that it is ensured that the wound dressing is neither changed too early nor too late. Further, by optimising the use of the wound dressing, the healing of the wound can be optimised, thereby providing improved well-being to the user.

A problem exists for an electronic device, e.g. a monitor device for monitoring an ostomy appliance or a wound dressing, in balancing, sometimes conflicting, requirements between medical device classification and certification, waterproofing, simplicity, usability etc. Thus, solutions towards balancing these requirements are of great interest.

Although the following discussion in particular relates to a monitor device for use with an ostomy appliance or a wound dressing, additional or alternative uses are foreseen within the scope of the invention.

WO 2013/184784 A1 may be regarded as the closest prior art, it relates to a portable medical device including an interface for accepting a power supply and enabling data transfer while still connected to a human body.

US 6 171 289 B1 relates to a disposable device for securing an ostomy bag to a stoma, the device comprising an adhesive seal and means for detecting wetness of the adhesive composition and means for triggering an alarm when a predetermined level of wetness is reached in the adhesive. WO 2007/098762 A1 relates to a method and dressing for detecting detachment of the dressing, which is applied to a surface of an at least partly electrically conductive object. The dressing comprises an adhesive and at least two electrodes arranged in a distance from the electrically conductive object. Changes in capacitance are detected, and an alarm is activated when the changes of the capacitance reach a predetermined value.

WO 2019/120428 A1 relates to a base plate for an ostomy appliance and related methods. The base plate comprises a monitor interface for forming mechanical and electrical connection with a monitor device.

US 9 020 601 B2 relates to methods, systems, and devices for receiving a data signal and a power signal by a hearing prosthesis. An input signal is received at a first unit of a hearing prosthesis. The first unit receives the input signal at a wired interface module that conforms to a standardized interface. The first unit identifies a data signal and a power signal included in the input signal. The first unit transfers at least a portion of the power signal to a second unit of the hearing prosthesis. The second unit is implanted in a body and is configured to stimulate an auditory organ, allowing a user to perceive at least a portion of a sound.

### SUMMARY

It is an object of the present disclosure to provide a medical device system comprising a base station and a medical device for being worn by a user, such as a monitor device for being connected to a sensor assembly.

The monitor device may be provided to facilitate detection of risk of failure and/or risk of leakage with respect to an adhesive base plate of an ostomy appliance or the monitor device may be provided to facilitate detection of exudate propagation or patterns of wound dressings. Furthermore, it is an object of the present disclosure to provide a medical device system, e.g. a base station and/or a monitor device, which is easy and intuitive to use, and which facilitates use and maintenance of the devices. Particularly, it is an object of the present disclosure to provide for easy and convenient use of the disclosed system, e.g. to avoid or reduce the occurrence of unintended or unsatisfactory situations.

Thus, the present disclosure relates to a medical device system and components thereof. Accordingly, a medical device system comprising a medical device and a base station is disclosed.

### BRIEF DESCRIPTION OF THE FIGURES

Embodiments of the disclosure will be described in more detail in the following with regard to the accompanying figures. The figures show one way of implementing the present invention and are not to be construed as being limiting to other possible embodiments falling within the scope of the attached claim set.
Fig. 1 schematically illustrates an exploded view of an exemplary adhesive base plate,
Fig. 2 schematically illustrates an exploded view of an exemplary adhesive base plate,
Fig. 3 schematically illustrates part of an exemplary adhesive base plate with an electrode assembly, and an exemplary monitor device,
Fig. 4 is a schematic block diagram of an exemplary monitor device,
Fig. 5 schematically illustrates an exemplary medical device system,
Fig. 6 schematically illustrates an exploded view of an exemplary base station,
Fig. 7 schematically illustrates an exploded view of an exemplary medical device, and
Fig. 8 shows a simplified block diagram of an exemplary base station.

### DETAILED DESCRIPTION

In the following Detailed Description, reference is made to the accompanying drawings, which form a part hereof, and in which is shown by way of illustration specific embodiments in which the invention may be practiced. In this regard, directional terminology, such as "top," "bottom," "front," "back," "leading," "trailing," etc., is used with respect to the orientation of the Figure(s) being described. Because components of embodiments can be positioned in a number of different orientations, the directional terminology is used for purposes of illustration and is in no way limiting. It is to be understood that other embodiments may be utilized, and structural or logical changes may be made without departing from the scope of the present invention. The following detailed description, therefore, is not to be taken in a limiting sense, and the scope of the present invention is defined by the appended claims.

It is to be understood that the features of the various exemplary embodiments described herein may be combined with each other, unless specifically noted otherwise.

Throughout this disclosure, the words "stoma" and "ostomy" are used to denote a surgically created opening bypassing the intestines or urinary tract system of a person. The words are used interchangeably, and no differentiated meaning is intended. The same applies for any words or phrases derived from these, e.g. "stomal", "ostomies" etc. Also, the solid and liquid wastes emanating from the stoma may be referred to as both stomal "output," "waste(s)," and "fluids" interchangeably. A subject having undergone ostomy surgery may be referred to as "ostomist" or "ostomate" - moreover, also as "patient" or "user". When applicable, "patient" or "user" may as well be used to describe a subject wearing a wound dressing. However, in some cases "user" may also relate or refer to a health care professional (HCP), such as a surgeon or an (ostomy care) nurse or others. In those cases, it will either be explicitly stated, or be implicit from the context that the "user" is not the "patient" him- or herself.

In the following, whenever referring to proximal side of a device or part of a device, the referral is to the skin-facing side, when an ostomy appliance or wound dressing is worn by a user. Likewise, whenever referring to the distal side of a device or part of a device, the referral is to the side facing away from the skin, when an ostomy appliance or wound dressing is worn by a user. In other words, the proximal side is the side closest to the user, when the appliance is fitted on a user and the distal side is the opposite side - the side furthest away from the user in use.

When considering an ostomy appliance, the axial direction is defined as the direction of the stoma, when the appliance is worn by a user. Thus, the axial direction is generally perpendicular to the skin or abdominal surface of the user. The latter definition further applies when considering wounds/wound dressings.

The radial direction is defined as transverse to the axial direction that is transversely to the direction of the stoma, i.e. "across" the distal/proximal surface of the base plate. In some sentences, the words "inner" and "outer" may be used. These qualifiers should generally be perceived with respect to the radial direction, such that a reference to an "outer" element means that the element is farther away from a centre portion of the ostomy appliance than an element referenced as "inner". In addition, "innermost" should be interpreted as the portion of a component forming a centre of the component and/or being adjacent to the centre of the component. In analogy, "outermost" should be interpreted as a portion of a component forming an outer edge or outer contour of a component and/or being adjacent to that outer edge or outer contour.

The use of the word "substantially" as a qualifier to certain features or effects in this disclosure is intended to simply mean that any deviations are within tolerances that would normally be expected by the skilled person in the relevant field.

The use of the word "generally" as a qualifier to certain features or effects in this disclosure is intended to simply mean - for a structural feature: that a majority or major portion of such feature exhibits the characteristic in question, and - for a functional feature or an effect: that a majority of outcomes involving the characteristic provide the effect, but that exceptionally outcomes do no provide the effect.

The use of the word "essentially" as a qualifier to certain structural and functional features or effects in this disclosure is used for emphasizing what is the most important focus of something or fact about something (i.e. a feature may have or fulfil a variety of effects, but when the disclosure discusses one effect as being "essentially" provided, this is the focus and the most important effect in relation to the disclosure).

Throughout the disclosure, the use of the terms "first", "second", "third", "fourth", "primary", "secondary", "tertiary" etc. does not imply any particular order or importance but are included merely to identify individual elements. Furthermore, the labelling of a first element does not imply the presence of a second element and vice versa.

Disclosed is a medical device, a base station, and a medical device system comprising the disclosed medical device and base station.

The medical device may be configured for being worn by a user. For example, the medical device may comprise an appliance interface configured for being coupled to and/or communicating with a patient worn device. For example, the medical device may be a monitor device for being coupled to an adhesive base plate or a wound dressing, and/or to an electrode assembly for being coupled to an adhesive base plate or a wound dressing. In embodiments, the disclosed medical device system may additionally comprise the adhesive base plate, e.g. comprising a backing layer, a first adhesive layer, an electrode assembly comprising a plurality of electrodes, and a monitor interface configured for coupling the adhesive base plate to the medical device. In embodiments, the disclosed medical device system may additionally comprise the wound dressing, e.g. comprising a backing layer, an absorbent core layer, a first adhesive layer, an electrode assembly comprising a plurality of electrodes, and a monitor interface configured for coupling the wound dressing to the medical device. The absorbent core layer may be arranged between the backing layer and the first adhesive layer of the wound dressing.

The medical device may be considered an electronic medical device, i.e. the medical device comprises electronics configured to perform certain tasks, such as monitoring/controlling an electrode assembly as exemplified above. The electrode assembly may comprise at least one sensor for sensing a characteristic, such as presence of liquid and/or moisture.

The medical device comprises a rechargeable battery for powering the medical device and a base interface comprising a plurality of device terminals including a first device terminal, a second device terminal and a third device terminal. The plurality of device terminals may include a fourth device terminal. The medical device may comprise a wireless communication interface for wirelessly communicating with an accessory device, such as a smartphone or a tablet.

The medical device may comprise a medical device housing, e.g. enclosing the components of the medical device. The housing may be watertight and/or may form a watertight enclosure for the internal components of the medical device, thereby protecting the internal components of the medical device from the potentially harsh environments and everyday use, e.g. showering. The medical device may comprise electronic circuitry, e.g. including a processing unit and/or one or more interfaces, e.g. including the wireless communication interface. The medical device may comprise a memory, e.g. for storing ostomy or wound data and/or parameters data based on the ostomy or wound data. The memory may be connected to the processing unit and/or to the one or more interfaces, such as the wireless communication interface.

The base station comprises an input button for receiving one or more touch inputs including a first touch input, a power unit for receiving power for powering the base station, and a device interface for coupling to the base interface of the medical device. The device interface comprises a plurality of base terminals for being coupled to the plurality of device terminals including a first base terminal for being coupled to the first device terminal, a second base terminal for being coupled to the second device terminal and a third base terminal for being coupled to the third device terminal. The plurality of base terminals may include a fourth base terminal configured to be coupled to the fourth device terminal.

The plurality of base terminals comprises a base charging pair for being coupled to a device charging pair of the plurality of device terminals. The base charging pair may comprise the first base terminal and the second base terminal. The device charging pair may comprise the first device terminal and the second device terminal.

The plurality of base terminals comprises a first base signal pair for being coupled to a first device signal pair of the plurality of device terminals. The first base signal pair may comprise the first base terminal and the third base terminal. The first device signal pair may comprise the first device terminal and the third device terminal.

The base station is configured to provide a charging voltage between/across the base charging pair for charging the rechargeable battery of the medical device. The charging voltage may between 3 and 15 volts, such as between 5 and 10 volts, such as 6.5 volts. The base station is further configured to, in accordance with receipt of the one or more touch inputs, alter a first base signal between the first base signal pair.

The medical device is configured to detect a first device signal between the first device signal pair. The medical device is further configured to, in accordance with the first device signal being indicative of the input button receiving the first touch input, initiate a first mode of the medical device. The first mode may be a pairing mode of the wireless communication interface, e.g. wherein the medical device. Alternatively, the first mode may be a data transmission mode of medical device, a charging mode of the medical device, an on-state of the medical device, or an off-state of the medical device. In accordance with the medical device being in an off-state the first mode may be an on-state of the medical device, and in accordance with the medical device being in an on-state the first mode is an off-state of the medical device.

Thereby is provided a medical device system capable of controlling functionalities of the medical device by means of a button arranged in the base station. Thereby, the medical device can be provided without buttons, such as to provide a medical device being particularly watertight, or to reduce the complexity of the electronics provided in the medical device. Thus, the base station and the medical device are configured to work in cooperation in order to alter/control functionalities of the medical device as such.

The first device signal pair and/or the first base signal pair may comprise a terminal, such as the third device terminal and/or the third base terminal, not forming part of the device charging pair and/or the base charging pair. Thereby, a signal, such as the first device signal and/or the first base signal need not influence the charging voltage and the charging of the rechargeable battery.

The one or more touch inputs to be received by the input button may include a second touch input. The first touch input and the second touch input may be differentiated, e.g. by their duration. For example, the first touch input may comprise holding the touch on the input button for less than a threshold duration. The second touch input may comprise holding the touch on the input button for more than the threshold duration. For example, the first touch input may be a touch held on the input button for more than a first primary threshold duration and less than a first secondary threshold duration. The second touch input may be a touch held on the input button for more than the first secondary threshold duration.

The medical device may be configured to, in accordance with the first device signal being indicative of the input button receiving the second touch input, initiate a second mode of the medical device. The second mode may be a reset mode of the medical device, e.g. wherein the medical device is reset to factory settings and/or wherein a storage memory of the medical device is erased, or the second mode may be a mode selected from the previously listed modes disclosed in relation to the first mode.

The power unit of the base station may comprise a socket, such as a USB type C socket, for receiving a connector for delivering power for powering the base station. Rated input power voltage of the input voltage of the power unit may be between 2 and 15 volts, such as between 3 and 10 volts, such as 5 volts. The charging voltage may be more than the rated input power voltage. The power unit may comprise a safety circuit configured to, in accordance with an input voltage of the power unit being above a voltage safety threshold, discontinue powering the base station. The voltage safety threshold may be between 5 and 15 V, such as between 6 and 10 V, such as 6.5 V. Thereby, the base station may satisfy requirements for medical equipment by limiting a possible erroneous voltage output to less than the voltage safety threshold.

The base station may comprise an indicator light. The indicator light may be configured to provide a visual indication based on a second base signal between a second base signal pair of the plurality of base terminals. The second base signal pair is configured to be coupled to a second device signal pair of the plurality of device terminals. The second base signal pair may comprise the first base terminal and the fourth base terminal. The second device signal pair may comprise the first device terminal and the fourth device terminal. The medical device may be configured to control the indicator light by altering a second device signal between the second device signal pair, e.g. such that the second base signal is altered when the second device signal pair is coupled to the second base signal pair. For example, the medical device may be configured to alter the second device signal (and the second base signal) according to an estimated charge of the rechargeable battery. Alternatively, the medical device may be configured to alter the second device signal (and the second base signal) according to a pairing status of the wireless communication interface. Thus, the medical device may be configured to control the indicator light of the base station. Thereby, characteristics such as charging status of the medical device can be communicated to the user through the base station. Thereby, the medical device does not need to comprise such indicator light.

The device interface of the base station may comprise a primary magnetic element. The base interface of the medical device may comprise a secondary magnetic element. The primary magnetic element and the secondary magnetic element may be configured to provide a magnetic attractive force between the base station and the medical device.

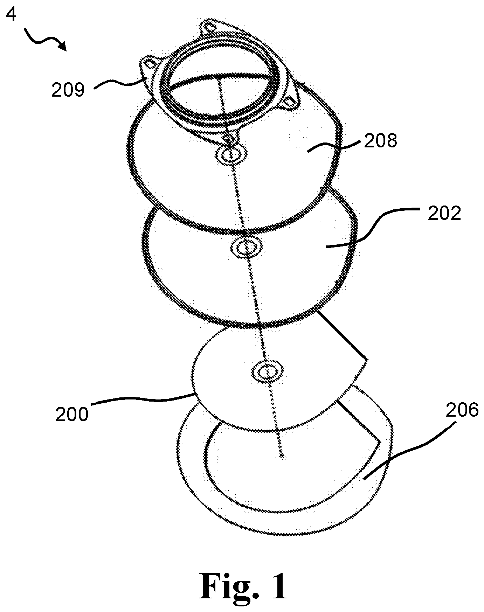

Fig. 1 schematically illustrates an exploded view of an exemplary adhesive base plate 4 of an ostomy appliance. The adhesive base plate 4 comprises a first adhesive layer 200, i.e. a first layer of an adhesive material. During use, a proximal surface of the first adhesive layer 200 adheres to the user's skin in the peristomal area and/or to additional seals, such as sealing paste, sealing tape and/or sealing ring. The adhesive base plate 4 optionally comprises a second adhesive layer 202, i.e. a second layer of an adhesive material, e.g. also denoted rim adhesive layer. The second adhesive layer 202 may be of a different adhesive material than the first adhesive layer 200. The adhesive base plate 4 may comprise a release liner 206, which may be peeled off by the user prior to applying the adhesive base plate 4 to the skin. The adhesive base plate 4 comprises a backing layer 208. The backing layer 208 is a protective layer protecting the adhesive layers, such as the first adhesive layer 200 and/or the second adhesive layer 202 from external strains and stress during use. Furthermore, the backing layer 208 also covers the adhesive layers, such as the first adhesive layer 200 and/or the second adhesive layer 202, such that the adhesive layers 200,202 does not adhere to clothes worn on top of the adhesive base plate 4.

The adhesive base plate 4 optionally comprises a coupling ring 209 for coupling an ostomy pouch to the adhesive base plate 4. Alternatively, e.g. for a one-part ostomy appliance, an ostomy pouch may be directly fastened to the adhesive base plate 4.

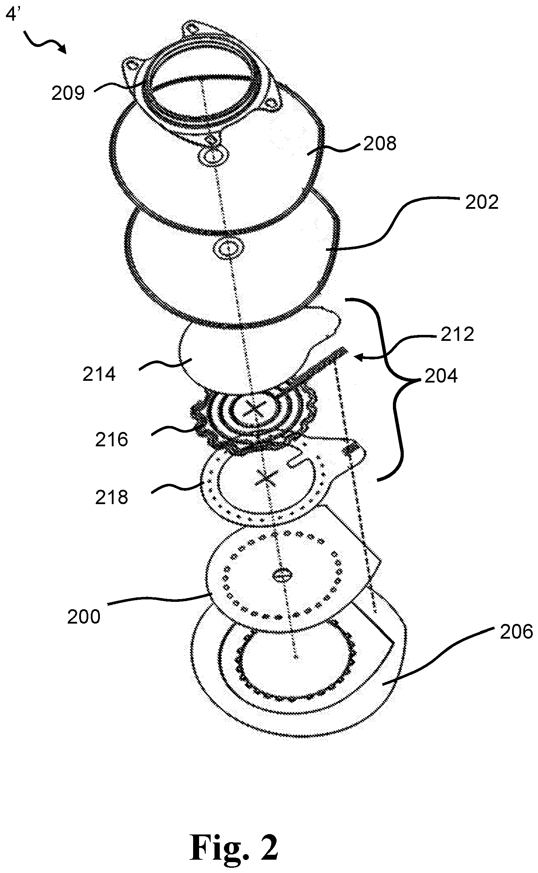

Fig. 2 schematically illustrates an exploded view of an exemplary adhesive base plate 4' of an ostomy appliance, wherein the adhesive base plate 4' is fitted with an electrode assembly 204, such as to enable electronic detection of increased risk of leakage. The adhesive base plate 4', like the adhesive base plate 4 of Fig. 1, comprises a first adhesive layer 200, an optional second adhesive layer 202. The adhesive base plate 4' may comprise a release liner 206. The adhesive base plate 4' may comprise a backing layer 208.

The adhesive base plate 4' comprises a plurality of electrodes 216 arranged in an electrode assembly 204. The electrode assembly 204 is arranged between the first adhesive layer 200 and the second adhesive layer 202. The electrode assembly 204 optionally comprises a support layer 214. The electrodes 216 may be formed on a proximal side of the support layer 214, such as by printing of conductive ink on the proximal side of the support layer 214. The electrode assembly 204 optionally comprises a masking element 218, e.g. covering or overlapping with parts of the electrodes 216 when seen in the axial direction, e.g. from a proximal side of the electrodes 216. The electrode assembly 204 forms a plurality of terminals 212 configured to form electrical connections with respective terminals of a monitor device, configured to be mechanically and/or electronically connected to the adhesive base plate 4'.

The adhesive base plate 4', as illustrated, is provided with an integrated electrode assembly 204. However, alternatively, the electrode assembly 204 may be provided as an additional device to be fitted to a (normal) adhesive base plate without such electrode assembly, such as the adhesive base plate 4 of Fig. 1. For example, an electrode assembly 204 may be attached to the adhesive side of the adhesive base plate 4, such as between the skin of the user and the first adhesive layer 200 of the adhesive base plate 4.

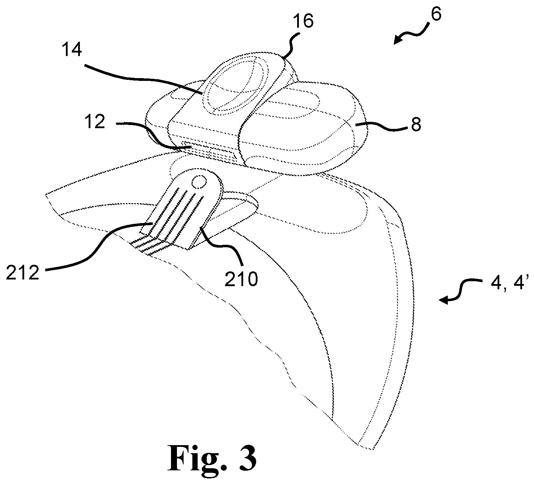

Fig. 3 schematically illustrates part of an exemplary adhesive base plate 4, 4' with an electrode assembly, and an exemplary monitor device 6. The adhesive base plate 4, 4' comprises a coupling element 210, to be received by the monitor device 6.

The monitor device 6 comprises a monitor device housing 8. The monitor device 6 comprises an interface opening 12, e.g. in a first rim part of the monitor device 6. The interface opening 12 is configured to receive the coupling element 210, such that the coupling element of the sensor assembly may be received by an appliance interface of the monitor device 6 through the interface opening 12.

The plurality of terminals 212 of the adhesive base plate 4, 4' may be provided on the coupling element 210. A plurality of corresponding terminals of the monitor device 6 may be provided inside the opening 12, such as to connect to the plurality of terminals 212 of the adhesive base plate 4, 4' when the monitor device 6 is coupled to the adhesive base plate 4, 4'.

The monitor device 6 may comprise a locking mechanism configured to lock the monitor device 6 in a coupled position with the adhesive base plate 4, 4'. The locking mechanism of the monitor device 6 may be configured to cooperate with the coupling element 210. The locking mechanism may comprise a locking element 14, e.g. a first button or a latch as illustrated. The locking element 14 may further comprise a locking element protrusion 16. The locking element 14 may be deflectable in a first direction and the locking element protrusion 16 may be configured for the user to pull/push the locking element 14 in an opposite second direction.

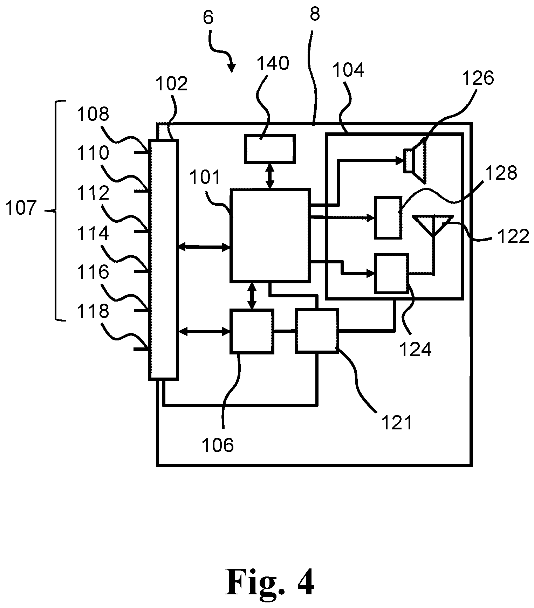

Fig. 4 is a schematic block diagram of an exemplary monitor device 6, such as the monitor device 6 as exemplified in relation to Fig. 3. The monitor device 6 comprises a monitor device housing 8. The monitor device 6 comprises electronic circuitry, including a processing unit 101 and one or more interfaces. The one or more interfaces includes an appliance interface 102 and an accessory interface 104. The monitor device 6 comprises memory 106 for storing ostomy data and/or parameters data based on the ostomy data. The memory 106 is connected to the processing unit 101 and/or the appliance interface 102.

The appliance interface 102 is configured for electrically and/or mechanically connecting the monitor device 6 to the ostomy appliance, such as to an electrode assembly of the ostomy appliance and/or an adhesive base plate comprising the electrode assembly. The appliance interface 102 may comprises a plurality of terminals 107 for forming electrical connections with respective terminals of the electrode assembly. The appliance interface 102 comprises a first terminal 108, a second terminal 110, a third terminal 112. The appliance interface optionally comprises a fourth terminal 114, a fifth terminal 116 and/or a sixth terminal 118. Alternatively, the appliance interface 102 may be configured for forming a wireless coupling with the electrode assembly.

The monitor device 6 comprises a device power unit 121 for powering the monitor device and active components thereof, i.e. the device power unit 121 is connected to the processing unit 101, the appliance interface 102, the accessory interface 104, and memory 106. The device power unit 121 comprises a rechargeable battery and charging circuitry. The charging circuitry is connected to the battery and terminals of the appliance interface 102 for charging the battery via terminals of the appliance interface 102.

The accessory interface 104 of the monitor device 6 is configured for connecting the monitor device 6 to one or more accessory devices such as a smart phone. The accessory interface 104 may comprise a wireless communication interface for wirelessly communicating with an accessory device. For example, the accessory interface 104 comprises an antenna 122 and a wireless transceiver 124 configured for wireless communication with accessory device(s). Optionally, the accessory interface 104 comprises a loudspeaker 126 and/or a haptic feedback element 128 for provision of respective audio signal and/or haptic feedback to the user.

The monitor device 6 may comprise a sensor unit 140 connected to the processing unit 101. The sensor unit 140 comprises a temperature sensor for feeding temperature data to the processor and a G-sensor or accelerometer for feeding acceleration data to the processing unit 101.

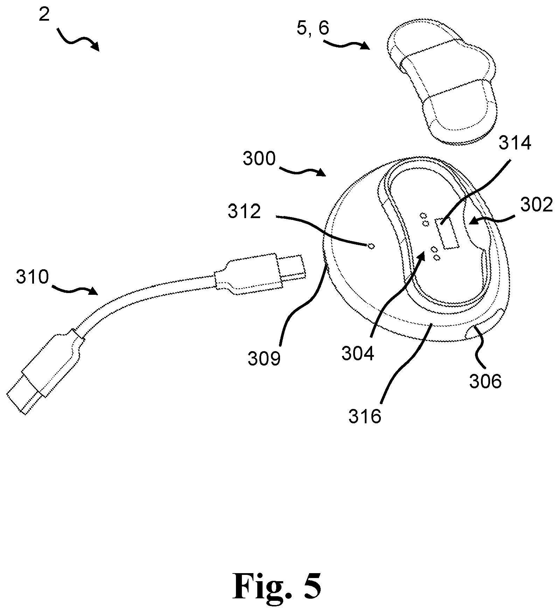

Fig. 5 schematically illustrates an exemplary medical device system 2 comprising a medical device 5, here illustrated as the exemplary monitor device 6 as described with respect to the previous figures. The medical device 5 is configured to be worn by a user, e.g. the monitor device 6 is configured to be coupled to an adhesive base plate being worn by an ostomist or to be coupled to a wound dressing. Thus, the medical device 5 may comprise an appliance interface, as described above, configured for being coupled to and/or communicating with a patient worn device, such as an adhesive base plate or a wound dressing. The medical device system 2 may comprise an adhesive base plate, such as the adhesive base plate 4, 4' of the previous figures, or wound dressing comprising an electrode assembly.

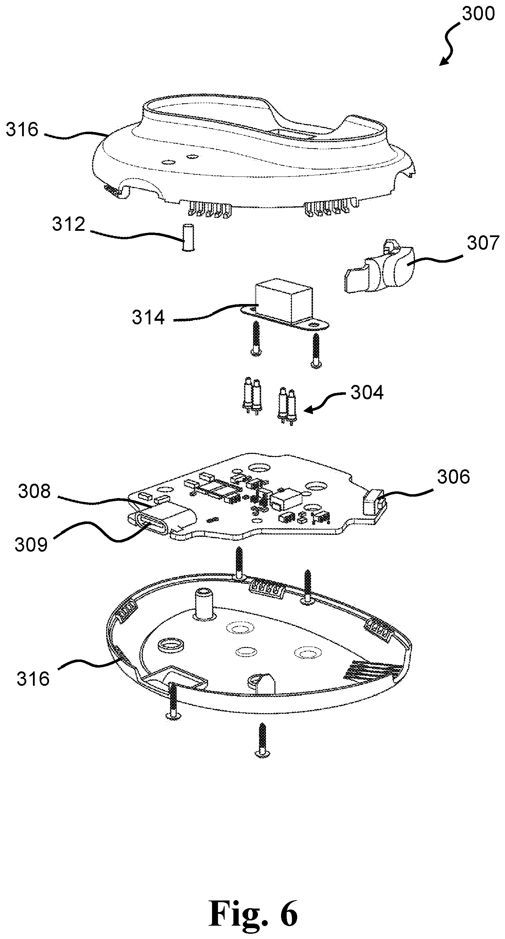

The medical device system 2 comprises a base station 300. An exploded view of the exemplary base station 300 is shown in Fig. 6. The base station 300 comprises an input button 306 for receiving touch inputs. The input button 306 may be covered by a button cover 307, e.g. provided in a flexible material, e.g. to provide a watertight input button. The base station 300 further comprises a power unit 308 (see Fig. 6) for receiving power for powering the base station 300. The power unit 308 may comprise a socket 309, such as a USB type C socket, for receiving a connector 310 for delivering power for powering the base station 300. The connector 310 may be configured to be connected to a standard USB-port on a PC or be connected to a USB power adaptor. The base station 300 further comprises a base housing 316. As illustrated in Fig. 6, the base housing may comprise upper and lower housing parts forming the base housing 316.

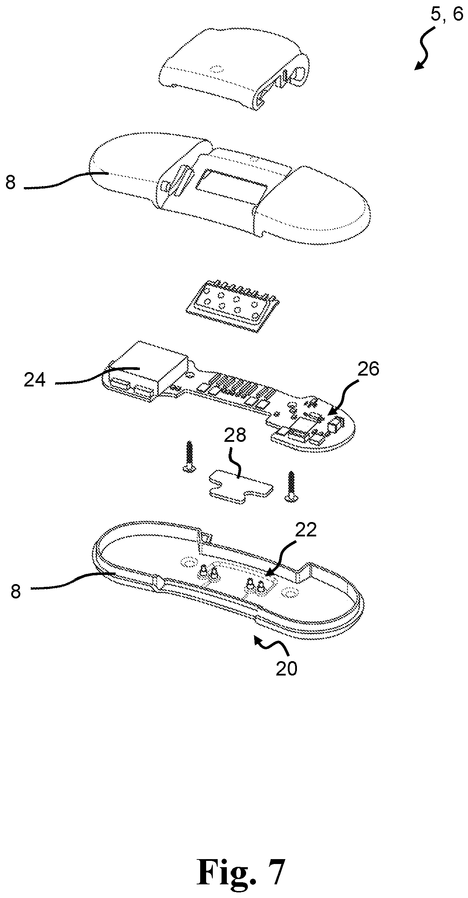

An exploded view of the exemplary medical device 5 is further schematically illustrated in Fig. 7. The medical device 5 comprises a base interface 20 comprising a plurality of device terminals 22 including a first device terminal, a second device terminal, a third device terminal, and an optional fourth device terminal. The device terminals 22 are configured to be coupled to respective base terminals 304 of the base station 300. The medical device 5 comprises a rechargeable battery 24 for powering the medical device 5, and a wireless communication interface for wirelessly communicating with an accessory device.

The base station 300 comprises a device interface 302 (see Fig. 5) for coupling to the base interface 20 of the medical device 5. The device interface 302 comprises a plurality of base terminals 304 for being coupled to the plurality of device terminals 22 of the medical device 5. The base terminals 304 include a first base terminal for being coupled to the first device terminal of the medical device 5, a second base terminal for being coupled to the second device terminal of the medical device 5, and a third base terminal for being coupled to the third device terminal of the medical device 5. In the illustrated example, the base terminals 304 include a fourth base terminal for being coupled to the fourth device terminal of the medical device 5. The plurality of base terminals 304 comprises a plurality of pairs of terminals for being coupled to corresponding pairs of terminals of the plurality of device terminals 22. For example, the plurality of base terminals 304 comprises a base charging pair for being coupled to a device charging pair of the medical device 5, and the base station is configured to provide a charging voltage between the base charging pair for charging the rechargeable battery 24 of the medical device 5. Furthermore, the plurality of base terminals 304 comprises a first base signal pair for being coupled to a first device signal pair of the medical device 5. In accordance with receipt of a touch input via the input button 306, the base station may alter a first base signal between the first base signal pair. For example, a voltage difference between the first base signal pair of the plurality of base terminals 304 may be set to 2 V if the user press and hold the input button 306 for a predetermined amount of time, and may be set to 4 V if the user press and hold the input button 306 for a second predetermined amount of time, etc.

The medical device 5 may be configured to detect the voltage difference between the first base signal pair of the plurality of base terminals 304 and/or between the first device signal pair of the plurality of device terminals 22 coupled to the first base signal pair. According to the detected voltage difference, the medical device 5 may activate a predefined mode in accordance with the detected voltage difference. Thereby, a touch input on the input button 306 may trigger different functionalities of a medical device 5 coupled to the base station 300. Thereby, means are provided for manipulating/controlling certain functionalities of the medical device 5 by means of the base station 300. Thereby, the medical device 5 can be provided without input buttons, e.g. to provide a particularly waterproof housing. In other words, the base station 300 allows for controlling certain functionalities of the medical device 5, whereby the medical device 5 can be provided without such means for controlling its functionalities.

For example, the medical device 5 may be configured to detect a first device signal between the first device signal pair, and in accordance with the first device signal being indicative of the input button receiving a first touch input (e.g. touch and hold for more than a first primary threshold duration and less than a first secondary threshold duration), the medical device 5 may initiate a first mode. For example, the first mode may be a pairing mode of the wireless communication interface of the medical device, e.g. in the pairing mode the medical device 5 may be discoverable via Bluetooth by other devices, such as an accessory device. Alternatively, the first mode may be a data transmission mode, e.g. wherein the medical device 5 offloads stored data, such as stored monitor data, to an accessory device. Alternatively, the first mode may be a charging mode of the medical device 5, e.g. wherein the medical device 5 is being charged. Alternatively, the first mode may be an on-state of the medical device or an off-state of the medical device 5.

Alternatively or additionally, the first device signal may be indicative of the input button receiving a second touch input (e.g. touch and hold for more than the first secondary threshold duration), and in accordance with the first device signal being indicative of the input button receiving the second touch input, the medical device 5 may initiate a second mode of the medical device 5. The second mode may be a reset mode, e.g. wherein the medical device is reset to factory settings and/or wherein a storage memory of the medical device is erased. Alternatively, the second mode may be one of the modes described in relation to the first mode.

The base station 300 may comprise an indicator light 312. The indicator light 312 may be configured to provide a visual indication based on the first base signal, or a second base signal between a second base signal pair of the plurality of base terminals 304. The second base signal pair is configured to be coupled to a second device signal pair of the plurality of device terminals 22 of the medical device 5. The medical device 5 may be configured to control the indicator light 312 by altering a second device signal between the second device signal pair. For example, the medical device 5 may be configured to alter the second device signal, and thereby the output of the indicator light 312, according to an estimated charge of the rechargeable battery, e.g. to indicate remaining battery level. For example, the output of the indicator light 312 may be altered by altering the colour and/or the frequency of the output by the indicator light.

The device interface 302 of the base station may comprise a primary magnetic element 314. The base interface of the medical device 5 may comprises a secondary magnetic element 28. Thereby a magnetic attractive force may be provided between the base station 300 and the medical device 5, such as to aid in correctly positioning the medical device 5 on the base station 300, and/or to maintain the medical device 5 in the correct position on the base station 300.

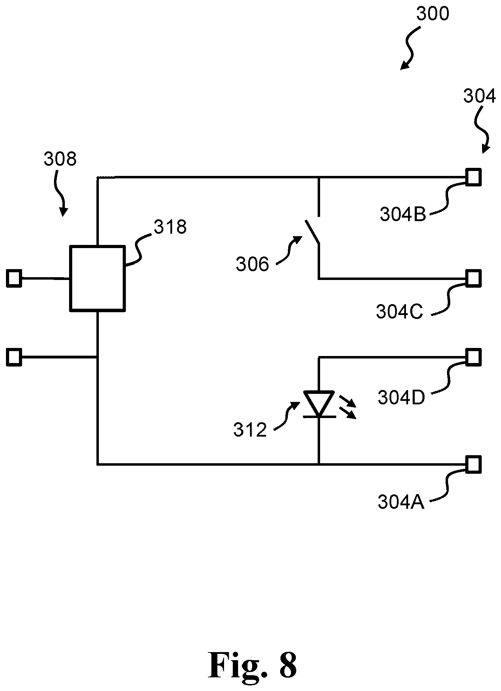

Fig. 8 shows a simplified block diagram of an exemplary base station 300, such as the base station 300 of the previous figures.

The base station 300 comprises, as part of a device interface for coupling the base interface of the medical device, a plurality of base terminals 304 including a first base terminal 304A, a second base terminal 304B, a third base terminal 304C, and a fourth base terminal 304D.

The base station 300 comprises a power unit 308 for receiving power, e.g. via USB type C connection, for powering the base station 300. The power unit 308 comprises a safety circuit 318. The safety circuit 318 is configured to discontinue power to the base station, in case an input voltage of the power unit 308 exceeds a voltage safety threshold. The voltage safety threshold may be between 5 and 15 V, such as between 6 and 10 V, such as 6.5 V.

The first base terminal 304A may be a ground terminal, e.g. the first base terminal 304A may be connected to a ground terminal of the power unit 308.

The first base terminal 304A and the second base terminal 304B forms a base charging pair for being coupled to a device charging pair of the plurality of device terminals, such as to allow charging of the rechargeable battery of the medical device.

The first base terminal 304A and the third base terminal 304C forms a first base signal pair for being coupled to a first device signal pair of the plurality of device terminals. The base station comprises an input button 306 for receiving touch inputs, resulting in a change of voltage difference between the first base signal pair.

The base station 300 comprises an indicator light 312, e.g. an LED. The indicator light is triggered based on a voltage difference between a second base signal pair of the plurality of base terminals 304 formed between the first base terminal 304A and the fourth base terminal 304D. Hence, the medical device being connected to the base station may output a voltage, such as the voltage of the second base terminal 304B on the fourth base terminal 304D, in which case the indicator light 312 will light up. Thus, the medical device is configured to control the indicator light 312 by altering the voltage between a second device signal pair being coupled to the second base signal pair.

Although the present disclosure is mainly focused towards a medical device, it is noted that the method and the principles as described herein may be used alternatively or additionally in a non-medical device, i.e. a device (not necessarily a medical device) comprising the features as described with respect to the mentioned medical device is also disclosed.

Although specific embodiments have been illustrated and described herein, it will be appreciated by those of ordinary skill in the art that a variety of alternate and/or equivalent implementations may be substituted for the specific embodiments shown and described without departing from the scope of the present invention. This application is intended to cover any adaptations or variations of body side members for ostomy appliances as discussed herein. Therefore, it is intended that this invention be limited only by the claims.

### REFERENCES

- 2: system
- 4, 4': adhesive base plate
- 5: medical device
- 6: monitor device
- 8: housing
- 12: opening
- 14: locking element
- 16: locking element protrusion
- 20: base interface
- 22: device terminals
- 24: battery
- 26: wireless communication interface
- 28: secondary magnetic element
- 101: processing unit
- 102: appliance interface
- 104: accessory interface
- 106: memory
- 107: terminals
- 108: first terminal of monitor device
- 110: second terminal of monitor device
- 112: third terminal of monitor device
- 114: fourth terminal of monitor device
- 116: fifth terminal of monitor device
- 118: sixth terminal of monitor device
- 121: device power unit
- 122: antenna
- 124: wireless transceiver
- 140: sensor unit
- 200: first adhesive layer
- 202: second adhesive layer
- 204: electrode assembly
- 206: release liner
- 208: backing layer
- 209: coupling ring
- 210: coupling element
- 212: terminals
- 214: support layer
- 216: electrodes
- 218: masking element
- 300: base station
- 302: device interface
- 304: base terminals
- 304A: first base terminal
- 304B: second base terminal
- 304C: third base terminal
- 304D: fourth base terminal
- 306: input button
- 307: button cover
- 308: power unit
- 309: socket
- 310: connector
- 312: indicator light
- 314: primary magnetic element
- 316: base housing
- 318: safety circuit

## Claims

1. A medical device system (2) comprising a medical device (5, 6) and a base station (300), the medical device (5, 6) being configured for being worn by a user, the medical device (5, 6) comprising:
- a base interface (20) comprising a plurality of device terminals (22) including a first device terminal, a second device terminal and a third device terminal,
- a rechargeable battery (24) for powering the medical device (5, 6),
the base station (300) comprising:
- a device interface (302) for coupling to the base interface (20) of the medical device (5, 6), the device interface (302) comprising a plurality of base terminals (304) for being coupled to the plurality of device terminals (22) and including a first base terminal (304A) for being coupled to the first device terminal, a second base terminal (304B) for being coupled to the second device terminal, and a third base terminal (304C) for being coupled to the third device terminal, the plurality of base terminals (304) comprising a base charging pair (304A, 304B) for being coupled to a device charging pair of the plurality of device terminals (22), the plurality of base terminals (304) comprising a first base signal pair (304A, 304C) for being coupled to a first device signal pair of the plurality of device terminals (2), and
- an input button (306) for receiving one or more touch inputs including a first touch input,
- a power unit (308) for receiving power for powering the base station (300),
the base station (300) being configured to:
- provide a charging voltage between the base charging pair (304A, 304B) for charging the rechargeable battery (24) of the medical device (5, 6), and
- in accordance with receipt of the one or more touch inputs, alter a first base signal between the first base signal pair (304A, 304C),
the medical device (5, 6) being configured to:
- detect a first device signal between the first device signal pair,
**characterised in that**
the medical device (5, 6) further being configured to:
- in accordance with the first device signal being indicative of the input button (306) receiving the first touch input, initiate a first mode of the medical device (5, 6).

2. Medical device system according to claim 1, wherein the medical device comprises a wireless communication interface (26) for wirelessly communicating with an accessory device.

3. Medical device system according to claim 2, wherein the first mode is a pairing mode of the wireless communication interface.

4. Medical device system according to any of claims 1-2, wherein the first mode is a data transmission mode of the medical device, a charging mode of the medical device, an on-state of the medical device, or an off-state of the medical device.

5. Medical device system according to any of claims 1-2, wherein in accordance with the medical device being in an off-state the first mode is an on-state of the medical device, and in accordance with the medical device being in an on-state the first mode is an off-state of the medical device.

6. Medical device system according to any of the preceding claims, wherein the base charging pair comprises the first base terminal (304A) and the second base terminal (304B), and the first base signal pair comprises the first base terminal (304A) and the third base terminal (304C), and/or wherein the device charging pair comprises the first device terminal and the second device terminal, and the first device signal pair comprises the first device terminal and the third device terminal.

7. Medical device system according to any of the preceding claims, wherein the power unit of the base station comprises a socket (309) for receiving a connector (310) for delivering power for powering the base station.

8. Medical device system according to any of the preceding claims, wherein the power unit of the base station comprises a safety circuit (318) configured to, in accordance with an input voltage of the power unit being above a voltage safety threshold, discontinue powering the base station.

9. Medical device system according to any of the preceding claims, wherein the base station comprises an indicator light (312), and wherein the indicator light is configured to provide a visual indication based on a second base signal between a second base signal pair of the plurality of base terminals, wherein the second base signal pair is configured to be coupled to a second device signal pair of the plurality of device terminals.

10. Medical device system according to claim 9, wherein the plurality of device terminals includes a fourth device terminal, and the plurality of base terminals includes a fourth base terminal (304D) configured to be coupled to the fourth device terminal, and wherein the second base signal pair (304A, 304D) comprises the first base terminal (304A) and the fourth base terminal (304D), and the second device signal pair comprises the first device terminal and the fourth device terminal.

11. Medical device system according to any of the preceding claims, wherein the device interface (302) of the base station comprises a primary magnetic element (314) and the base interface of the medical device comprises a secondary magnetic element (28), the primary magnetic element and the secondary magnetic element being configured to provide a magnetic attractive force between the base station and the medical device.

12. Medical device system according to any of the preceding claims, wherein the one or more touch inputs includes a second touch input, and the medical device being configured to, in accordance with the first device signal being indicative of the input button receiving the second touch input, initiate a second mode of the medical device.

13. Medical device system according to claim 12, wherein the second mode is a reset mode of the medical device.

14. Medical device system according to any of claims 12-13, wherein the first touch input comprises holding the touch on the input button for less than a threshold duration and the second touch input comprises holding the touch on the input button for more than the threshold duration.

15. Medical device system according to any of the preceding claims, wherein the medical device comprises an appliance interface (102) configured for being coupled to and/or communicating with a patient worn device.

## Patentansprüche

1. Medizinisches Vorrichtungssystem (2), das eine medizinische Vorrichtung (5, 6) und eine Basisstation (300) umfasst, wobei die medizinische Vorrichtung (5, 6) dazu ausgelegt ist, von einem Benutzer getragen zu werden, wobei die medizinische Vorrichtung (5, 6) Folgendes umfasst:
- eine Basisschnittstelle (20), die mehrere Vorrichtungsanschlüsse (22) umfasst, die einen ersten Vorrichtungsanschluss, einen zweiten Vorrichtungsanschluss und einen dritten Vorrichtungsanschluss beinhalten,
- eine wiederaufladbare Batterie (24) zum Versorgen der medizinischen Vorrichtung (5, 6) mit Leistung,
wobei die Basisstation (300) Folgendes umfasst:
- eine Vorrichtungsschnittstelle (302) zum Koppeln mit der Basisschnittstelle (20) der medizinischen Vorrichtung (5, 6), wobei die Vorrichtungsschnittstelle (302) mehrere Basisanschlüsse (304) umfasst, die zum Koppeln mit den mehreren Vorrichtungsanschlüssen (22) dienen und einen ersten Basisanschluss (304A), der zum Koppeln mit dem ersten Vorrichtungsanschluss dient, einen zweiten Basisanschluss (304B), der zum Koppeln mit dem zweiten Vorrichtungsanschluss dient, und einen dritten Basisanschluss (304C), der zum Koppeln mit dem dritten Vorrichtungsanschluss dient, beinhalten, wobei die mehreren Basisanschlüsse (304) ein Basisladepaar (304A, 304B) umfassen, das zum Koppeln mit einem Vorrichtungsladepaar der mehreren Vorrichtungsanschlüsse (22) dient, wobei die mehreren Basisanschlüsse (304) ein erstes Basissignalpaar (304A, 304C) umfassen, das zum Koppeln mit einem ersten Vorrichtungssignalpaar der mehreren Vorrichtungsanschlüsse (2) dient, und
- eine Eingabetaste (306) zum Empfangen einer oder mehrerer Berührungseingaben, die eine erste Berührungseingabe beinhalten,
- eine Leistungseinheit (308) zum Empfangen von Leistung zum Versorgen der Basisstation (300) mit Leistung,
wobei die Basisstation (300) zu Folgendem ausgelegt ist:
- Bereitstellen einer Ladespannung zwischen dem Basisladepaar (304A, 304B) zum Laden der wiederaufladbaren Batterie (24) der medizinischen Vorrichtung (5, 6), und
- in Übereinstimmung mit einem Empfang der einen oder der mehreren Berührungseingaben, Wechseln eines ersten Basissignals zwischen dem ersten Basissignalpaar (304A, 304C),
wobei die medizinische Vorrichtung (5, 6) zu Folgendem ausgelegt ist:
- Detektieren eines ersten Vorrichtungssignals zwischen dem ersten Vorrichtungssignalpaar,
**dadurch gekennzeichnet, dass** die medizinische Vorrichtung (5, 6) ferner zu Folgendem ausgelegt ist:
- in Übereinstimmung damit, dass das erste Vorrichtungssignal anzeigt, dass die Eingabetaste (306) die erste Berührungseingabe empfängt, Initiieren eines ersten Modus der medizinischen Vorrichtung (5, 6).

2. Medizinisches Vorrichtungssystem nach Anspruch 1, wobei die medizinische Vorrichtung eine drahtlose Kommunikationsschnittstelle (26) zum drahtlosen Kommunizieren mit einer Zubehörvorrichtung umfasst.

3. Medizinisches Vorrichtungssystem nach Anspruch 2, wobei der erste Modus ein Paarungsmodus der drahtlosen Kommunikationsschnittstelle ist.

4. Medizinisches Vorrichtungssystem nach einem der Ansprüche 1-2, wobei der erste Modus ein Datenübertragungsmodus der medizinischen Vorrichtung, ein Lademodus der medizinischen Vorrichtung, ein Einschaltzustand der medizinischen Vorrichtung oder ein Ausschaltzustand der medizinischen Vorrichtung ist.

5. Medizinisches Vorrichtungssystem nach einem der Ansprüche 1-2, wobei in Übereinstimmung damit, dass sich die medizinische Vorrichtung in einem Ausschaltzustand befindet, der erste Modus ein Einschaltzustand der medizinischen Vorrichtung ist, und in Übereinstimmung damit, dass sich die medizinische Vorrichtung in einem Einschaltzustand befindet, der erste Modus ein Ausschaltzustand der medizinischen Vorrichtung ist.

6. Medizinisches Vorrichtungssystem nach einem der vorhergehenden Ansprüche, wobei das Basisladepaar den ersten Basisanschluss (304A) und den zweiten Basisanschluss (304B) umfasst und das erste Basissignalpaar den ersten Basisanschluss (304A) und den dritten Basisanschluss (304C) umfasst, und/oder wobei das Vorrichtungsladepaar den ersten Vorrichtungsanschluss und den zweiten Vorrichtungsanschluss umfasst und das erste Vorrichtungssignalpaar den ersten Vorrichtungsanschluss und den dritten Vorrichtungsanschluss umfasst.

7. Medizinisches Vorrichtungssystem nach einem der vorhergehenden Ansprüche, wobei die Leistungseinheit der Basisstation eine Buchse (309) zum Empfangen eines Verbinders (310) zum Liefern von Leistung zum Versorgen der Basisstation mit Leistung umfasst.

8. Medizinisches Vorrichtungssystem nach einem der vorhergehenden Ansprüche, wobei die Leistungseinheit der Basisstation eine Sicherheitsschaltung (318) umfasst, die dazu ausgelegt ist, in Übereinstimmung damit, dass eine Eingangsspannung der Leistungseinheit über einer Spannungssicherheitsschwelle liegt, ein Versorgen der Basisstation mit Leistung zu unterbrechen.

9. Medizinisches Vorrichtungssystem nach einem der vorhergehenden Ansprüche, wobei die Basisstation eine Anzeigeleuchte (312) umfasst, und wobei die Anzeigeleuchte dazu ausgelegt ist, eine visuelle Anzeige basierend auf einem zweiten Basissignal zwischen einem zweiten Basissignalpaar der mehreren Basisanschlüsse bereitzustellen, wobei das zweite Basissignalpaar dazu ausgelegt ist, mit einem zweiten Vorrichtungssignalpaar der mehreren Vorrichtungsanschlüsse gekoppelt zu sein.

10. Medizinisches Vorrichtungssystem nach Anspruch 9, wobei die mehreren Vorrichtungsanschlüsse einen vierten Vorrichtungsanschluss beinhalten und die mehreren Basisanschlüsse einen vierten Basisanschluss (304D) beinhalten, der dazu ausgelegt ist, mit dem vierten Vorrichtungsanschluss gekoppelt zu sein, und wobei das zweite Basissignalpaar (304A, 304D) den ersten Basisanschluss (304A) und den vierten Basisanschluss (304D) umfasst und das zweite Vorrichtungssignalpaar den ersten Vorrichtungsanschluss und den vierten Vorrichtungsanschluss umfasst.

11. Medizinisches Vorrichtungssystem nach einem der vorhergehenden Ansprüche, wobei die Vorrichtungsschnittstelle (302) der Basisstation ein primäres magnetisches Element (314) umfasst und die Basisschnittstelle der medizinischen Vorrichtung ein sekundäres magnetisches Element (28) umfasst, wobei das primäre magnetische Element und das sekundäre magnetische Element dazu ausgelegt sind, eine magnetische Anziehungskraft zwischen der Basisstation und der medizinischen Vorrichtung bereitzustellen.

12. Medizinisches Vorrichtungssystem nach einem der vorhergehenden Ansprüche, wobei die eine oder die mehreren Berührungseingaben eine zweite Berührungseingabe beinhalten und die medizinische Vorrichtung dazu ausgelegt ist, in Übereinstimmung damit, dass das erste Vorrichtungssignal anzeigt, dass die Eingabetaste die zweite Berührungseingabe empfängt, einen zweiten Modus der medizinischen Vorrichtung zu initiieren.

13. Medizinisches Vorrichtungssystem nach Anspruch 12, wobei der zweite Modus ein Rücksetzmodus der medizinischen Vorrichtung ist.

14. Medizinisches Vorrichtungssystem nach einem der Ansprüche 12-13, wobei die erste Berührungseingabe ein Halten der Berührung an der Eingabetaste für weniger als eine Schwellendauer umfasst und die zweite Berührungseingabe ein Halten der Berührung an der Eingabetaste für mehr als die Schwellendauer umfasst.

15. Medizinisches Vorrichtungssystem nach einem der vorhergehenden Ansprüche, wobei die medizinische Vorrichtung eine Geräteschnittstelle (102) umfasst, die dazu ausgelegt ist, mit einer von einem Patienten getragenen Vorrichtung gekoppelt zu sein und/oder mit dieser zu kommunizieren.

## Revendications

1. Système de dispositif médical (2) comprenant un dispositif médical (5, 6) et une station de base (300), le dispositif médical (5, 6) étant configuré pour être porté par un utilisateur, le dispositif médical (5, 6) comprenant :
- une interface de base (20) comprenant une pluralité de bornes de dispositif (22) comportant une première borne de dispositif, une deuxième borne de dispositif et une troisième borne de dispositif,
- une batterie rechargeable (24) permettant d'alimenter le dispositif médical (5, 6),
la station de base (300) comprenant :
- une interface de dispositif (302) pour un couplage avec l'interface de base (20) du dispositif médical (5, 6), l'interface de dispositif (302) comprenant une pluralité de bornes de base (304) destinées à être couplées à la pluralité de bornes de dispositif (22) et comportant une première borne de base (304A) destinée à être couplée à la première borne de dispositif, une deuxième borne de base (304B) destinée à être couplée à la deuxième borne de dispositif, et une troisième borne de base (304C) destinée à être couplée à la troisième borne de dispositif, la pluralité de bornes de base (304) comprenant une paire de charge de base (304A, 304B) destinée à être couplée à une paire de charge de dispositif de la pluralité de bornes de dispositif (22), la pluralité de bornes de base (304) comprenant une première paire de signaux de base (304A, 304C) destinée à être couplée à une première paire de signaux de dispositif de la pluralité de bornes de dispositif (2), et
- un bouton d'entrée (306) pour recevoir une ou plusieurs entrées tactiles comportant une première entrée tactile,
- une unité d'alimentation (308) pour recevoir de la puissance pour alimenter la station de base (300),
la station de base (300) étant configurée pour :
- fournir une tension de charge entre la paire de charge de base (304A, 304B) pour charger la batterie rechargeable (24) du dispositif médical (5, 6), et
- en fonction de la réception de la ou des entrées tactiles, modifier un premier signal de base entre la première paire de signaux de base (304A, 304C),
le dispositif médical (5, 6) étant configuré pour :
- détecter un premier signal de dispositif entre la première paire de signaux de dispositif,
**caractérisé en ce que** le dispositif médical (5, 6) est en outre configuré pour :
- en fonction du premier signal de dispositif indiquant que le bouton d'entrée (306) reçoit la première entrée tactile, lancer un premier mode du dispositif médical (5, 6).

2. Système de dispositif médical selon la revendication 1, le dispositif médical comprenant une interface de communication sans fil (26) pour communiquer sans fil avec un dispositif accessoire.

3. Système de dispositif médical selon la revendication 2, le premier mode étant un mode d'appariement de l'interface de communication sans fil.

4. Système de dispositif médical selon l'une quelconque des revendications 1 et 2, le premier mode étant un mode de transmission de données du dispositif médical, un mode de charge du dispositif médical, un état sous tension du dispositif médical, ou un état hors tension du dispositif médical.

5. Système de dispositif médical selon l'une quelconque des revendications 1 et 2, en fonction du fait que le dispositif médical est dans un état hors tension, le premier mode étant un état sous tension du dispositif médical, et en fonction du fait que le dispositif médical est dans un état sous tension, le premier mode étant un état hors tension du dispositif médical.

6. Système de dispositif médical selon l'une quelconque des revendications précédentes, la paire de charge de base comprenant la première borne de base (304A) et la deuxième borne de base (304B), et la première paire de signaux de base comprenant la première borne de base (304A) et la troisième borne de base (304C), et/ou la paire de charge de dispositif comprenant la première borne de dispositif et la deuxième borne de dispositif, et la première paire de signaux de dispositif comprenant la première borne de dispositif et la troisième borne de dispositif.

7. Système de dispositif médical selon l'une quelconque des revendications précédentes, l'unité d'alimentation de la station de base comprenant une prise (309) pour recevoir un connecteur (310) pour fournir de la puissance pour alimenter la station de base.

8. Système de dispositif médical selon l'une quelconque des revendications précédentes, l'unité d'alimentation de la station de base comprenant un circuit de sécurité (318) configuré pour, en fonction d'une tension d'entrée de l'unité d'alimentation supérieure à un seuil de sécurité de tension, interrompre l'alimentation de la station de base.

9. Système de dispositif médical selon l'une quelconque des revendications précédentes, la station de base comprenant un voyant lumineux (312), et le voyant lumineux étant configuré pour fournir une indication visuelle sur la base d'un deuxième signal de base entre une deuxième paire de signaux de base de la pluralité de bornes de base, la deuxième paire de signaux de base étant configurée pour être couplée à une deuxième paire de signaux de dispositif de la pluralité de bornes de dispositif.

10. Système de dispositif médical selon la revendication 9, la pluralité de bornes de dispositif comprenant une quatrième borne de dispositif, et la pluralité de bornes de base comprenant une quatrième borne de base (304D) configurée pour être couplée à la quatrième borne de dispositif, et la deuxième paire de signaux de base (304A, 304D) comprenant la première borne de base (304A) et la quatrième borne de base (304D), et la deuxième paire de signaux de dispositif comprenant la première borne de dispositif et la quatrième borne de dispositif.

11. Système de dispositif médical selon l'une quelconque des revendications précédentes, l'interface de dispositif (302) de la station de base comprenant un élément magnétique primaire (314) et l'interface de base du dispositif médical comprenant un élément magnétique secondaire (28), l'élément magnétique primaire et l'élément magnétique secondaire étant configurés pour fournir une force d'attraction magnétique entre la station de base et le dispositif médical.

12. Système de dispositif médical selon l'une quelconque des revendications précédentes, la ou les entrées tactiles comprenant une deuxième entrée tactile, et le dispositif médical étant configuré pour, en fonction du premier signal de dispositif indiquant que le bouton d'entrée reçoit la deuxième entrée tactile, lancer un deuxième mode du dispositif médical.

13. Système de dispositif médical selon la revendication 12, le deuxième mode étant un mode de remise à zéro du dispositif médical.

14. Système de dispositif médical selon l'une quelconque des revendications 12 et 13, la première entrée tactile comprenant le maintien du contact sur le bouton d'entrée pendant moins d'une durée de seuil et la deuxième entrée tactile comprenant le maintien du contact sur le bouton d'entrée pendant plus que la durée de seuil.

15. Système de dispositif médical selon l'une quelconque des revendications précédentes, le dispositif médical comprenant une interface d'appareil (102) configurée pour être couplée à et/ou communiquer avec un dispositif porté par un patient.
